# EUROPEAN PATENT APPLICATION

(11) **EP 3 771 744 A1**
(43) Date of publication of application: **03.02.2021**
(21) Application number: 20187830.3
(22) Date of filing: 27.07.2020
(51) Int. Cl.: C12N 5/0783, A61K 35/17, C12N 5/078

(54) **A METHOD FOR PRODUCING ANTIGEN-SPECIFIC T CELLS**

(30) Priority: 29.07.2019 JP 2019138480
(71) Applicant: Thyas Co. Ltd., Sakyo-ku Kyoto 606-8501 (JP)
(72) Inventor: YASUI, Yutaka, Kyoto, 606-8501 (JP)
(74) Representative: De Clercq & Partners

(57) **Abstract**

The present invention relates to a method for producing antigen-specific T cells, the antigen-specific T cells produced by the method, and a pharmaceutical composition containing the antigen-specific T cells.

## Description

### TECHNICAL FIELD

The present invention relates to a method for producing antigen-specific T cells, the antigen-specific T cells produced by the method, and a pharmaceutical composition comprising the antigen-specific T cells.

### TECHNICAL BACKGROUND

T cells play a central role in immune responses to foreign pathogens such as bacteria or virus, or abnormal cells such as cancer cells. It is accordingly considered that decrease in T cell function contributes to pathogen infection and development of cancer. For a disease caused by decrease in T cell function, supplementation and regeneration of T cells can be extremely effective means for improving pathological condition or treating the disease.

As a therapeutic method for cancer and infectious disease, a method of amplifying T cells in-vitro and returning them back to the patient is conducted. In order to carry out the therapeutic method, target antigen-specific T cells are required. Cancer-specific T cells also present in peripheral blood. However, T cells which are infiltrating in tumor are highly specific to cancer.

### SUMMARY OF THE INVENTION

### Problems to be solved by the invention

Among the T cells presenting in peripheral blood and infiltrating in tumor tissue, very few of them are specific to target antigen. Therefore, in order to obtain target antigen-specific T cells, it is necessary to carry out amplification culture by stimulation with target-antigenic peptide or protein. However, since the amplification efficiency of target antigen-specific T cells is low and the number of cells obtained is small, the therapeutic effect is also limited. Accordingly, development of a method for efficiently amplifying and separating target antigen-specific T cells is desired.

The present inventor conducted diligent research to find a method for efficiently amplifying and separating target antigen-specific T cells. He found out a method for that by stimulating mononuclear cells in a medium containing cytotoxic inhibitor, and completed the present invention after further study.

### Means for solving the problems

The object of the present invention is achieved by following inventions.
(1) A method for producing antigen-specific T cells, characterized by comprising a step of stimulating mononuclear cells in a medium containing cytotoxic inhibitor.
(2) The method according to (1), wherein the cytotoxic inhibitor is perforin inhibitor or granzyme inhibitor.
(3) The method according to (2), wherein the perforin inhibitor is one or more selected from the group consisting of Concanamycin A, SN34960, diarylthiophene, ethylene glycol bis (β-aminoethylether)- N,N,N',N'-tetraacetic acid (EGTA), ethylenediamine tetraacetic acid (EDTA), 1,2-bis-(o-aminophenoxy)-ethane-N,N,N',N'-tetraacetic acid (BAPTA), N-(2-hydroxyethyl)ethylenediamine-N,N',N'-triacetic acid (HEDTA) and nitrilotriacetic acid (NTA).
(4) The method according to (3), wherein the perforin inhibitor is Concanamycin A.
(5) The method according to any one of (1) to (4), wherein the mononuclear cells are lymphocyte.
(6) The method according to any one of (1) to (5), wherein the mononuclear cells are isolated from peripheral blood or tumor.
(7) The method according to any one of (1) to (6), wherein the antigen is tumor-related or variant antigen, or viral antigen.
(8) The method according to (7), wherein the tumor-related or variant antigen is peptide cancer antigen.
(9) The method according to (7), wherein the viral antigen is inactivated HBV or its virus-derived protein, or inactivated HPV or its virus-derived protein.
(10) The method according to any one of (1) to (9), wherein the mononuclear cells are stimulated with antigen.
(11) The method according to any one of (1) to (10), wherein the antigen is one or more peptide cancer antigens selected from the group consisting of GPC3, WT1, XAGE1, LMP2 and neoantigen, or one or more viral antigens selected from the group consisting of EBV LMP1, EBV LMP2, EBNA (EBV nuclear antigen), HPV E1, HPV E2, HPV E6, HPV E7, HBV HBs, HBV HBc, HBV HBe, HCV core, HCV NS3, HCV NS3/4A, HCV E1, HCV E2 , HCV NS5A, HIV gag, HIV pol, HIV nef, HIV env, CMV pp65, Influenza M1, HTLV-1 Ta and HBZ.
(12) Antigen-specific T cells obtained by the method according to any one of (1) to (11).
(13) A pharmaceutical composition containing the antigen-specific T cells according to (12).
(14) The pharmaceutical composition according to (13) for preventing or treating cancer or viral infection.
(15) The pharmaceutical composition according to (14), wherein the cancer is selected from the group consisting of ovarian cancer, hepatoblastoma, liver cancer, gastric cancer, esophageal cancer, pancreatic cancer, renal cell cancer, breast cancer, malignant melanoma, non-small cell lung cancer, cervical cancer, glioblastoma, prostate cancer, neuroblastoma, chronic lymphocytic leukemia, papillary thyroid cancer, colon cancer, and B-cell non-Hodgkin lymphoma.
(16) The pharmaceutical composition according to (14), wherein the viral infection is selected from the group consisting of hepatitis B virus, human papilloma virus, hepatitis C virus, EB virus, human immunodeficiency virus (HIV), human cytomegalovirus (CMV), influenza virus and human T-cell leukemia virus (HTLV).
(17) A kit for preventing or treating cancer or viral infection, which comprises the pharmaceutical composition according to any one of (13) to (16).
(18) A preventive or therapeutic agent for cancer or viral infection, which comprises the pharmaceutical composition according to any one of (13) to (16).
(19) A method for preventing or treating cancer or viral infection, characterized by administering the pharmaceutical composition according to any one of (13) to (16) to a patient.
(20) A method for preventing or treating cancer or viral infection, characterized by administering the antigen-specific T cells to a patient, which comprises a step of isolating mononuclear cells from peripheral blood, a step of stimulating the isolated mononuclear cells in a medium containing cytotoxic inhibitor, and a step of separating antigen-specific T cells from the mononuclear cells obtained by the stimulation.
(21) A pharmaceutical composition comprising antigen-specific T cells, wherein the T cells are (i) CD3 and CD45 positive, (ii) proliferative, and (iii) could release IFN-γ and Granzyme B upon activation by antigen stimulation.
(22) The pharmaceutical composition according to (21), wherein the percentage of the number of antigen-specific T cells to the total number of T cells in the pharmaceutical composition is 10% or more.
(23) The pharmaceutical composition according to (22), wherein the number of the antigen-specific T cells is 1 × 10³ or more.

### EFFECTS OF THE INVENTION

According to the present invention, target antigen-specific T cells can be identified by expressing activation marker when stimulated with a target-antigenic peptide or protein.

According to the present invention, the percentage and cell number of activation marker-expressing cells (CD137) after amplification culture were increased. As the activation marker, CD137(4-1BB), IFN-g, CD107a and the like are used.

Target antigen-specific T cells are obtained efficiently according to the present invention, so that it is possible to reduce the amount of necessary raw materials (peripheral blood or tumor).

Target antigen-specific T cells are obtained efficiently according to the present invention, so that the number of cells transplanted can be increased, and improvement in therapeutic effect can be expected.

According to the present invention, regenerated T cells can be produced efficiently via iPS cells using the target antigen-specific T cells obtained.

### BRIEF DESCRIPTION OF FIGURES

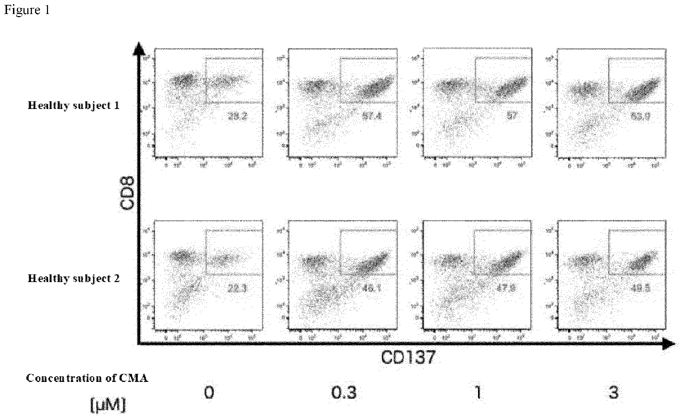

Figure 1 shows the percentage of T cells activated by antigenic peptide stimulation. It was revealed that the percentage of CD137+ T cells activated with EBV-LMP2A peptide (viral antigen) was increased by addition of Concanamycin A (CMA) in two samples of mononuclear cells from peripheral blood of a healthy subject.

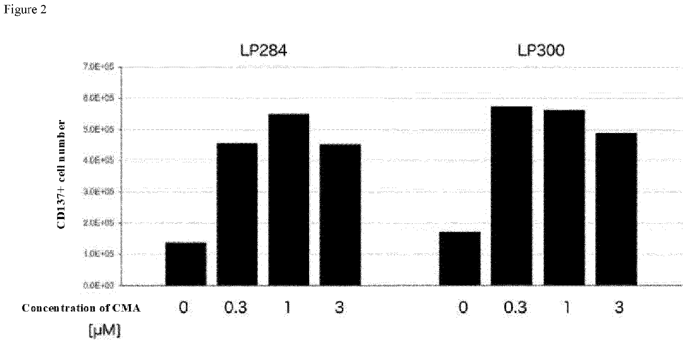

Figure 2 shows the number of T cells activated by antigenic peptide stimulation. It was revealed that the number of CD137+ T cells activated with EBV-LMP2A peptide was increased by addition of CMA in two samples of mononuclear cells from peripheral blood of a healthy subject.

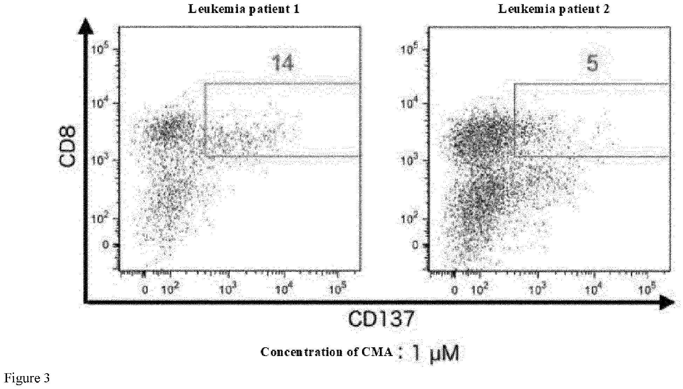

Figure 3 shows the percentage of T cells activated by antigenic peptide stimulation. CD137-expressing T cells are induced by activation with WT1 (cancer-related antigen) peptide in a medium added with 1 µM CMA in two samples of mononuclear cells from peripheral blood of a leukemia patient.

### EMBODIMENTS FOR CARRYING OUT THE INVENTION

The present invention provides a method for efficient production of target antigen-specific T cells. The method is characterized by comprising a step of stimulating mononuclear cells in a medium containing cytotoxic inhibitor.

The present invention provides a method for producing antigen-specific T cells, which comprises specifically a step of isolating mononuclear cells from peripheral blood, a step of stimulating the isolated mononuclear cells in a medium containing cytotoxic inhibitor, and a step of separating antigen-specific T cells from the mononuclear cells obtained by the stimulation.

The T cells in the present invention are derived preferably from mammals, more preferably from humans (cancer patients or non-cancer patients). It is preferable that the cancer or non-cancer patients have been administered, or are currently being administered, or are scheduled to be administered in the future with cancer vaccine. The cancer vaccine to be administered may be one or more types. Examples of T cells include, but are not limited thereto, αβ T cells, γδ T cells, helper T cells, regulatory T cells, cytotoxic T cells, and natural killer (NK) T cells. In addition, as a collection source of T cells, peripheral blood is preferable, because it is less invasive. However, it is not limited thereto. Examples of other preferable collection sources include cancer tissue or tumor tissue or other tissue or organ, or blood, cord blood, lymph fluid, interstitial fluid (interstitial fluid, intercellular fluid and interstitial fluid), body cavity fluid (ascites fluid, pleural fluid, pericardial effusion, cerebrospinal fluid, joint fluid and aqueous humor), nasal discharge, urine and all other collection sources in the body. In an embodiment of the invention, preferable T cells are derived from tumor tissue. T cells derived from tumor tissue are usually tumor infiltrating T cells.

A "cancer vaccine" is a composition comprising vaccine antigen, which is cancer- or tumor-specific protein or peptide, and is derived from cancer- or tumor-related antigen, for inducing cancer- or tumor-specific immune responses. Cellular vaccine such as dendritic cells which are antigen pulsed with cancer tissue, or protein or peptide which are cancer- or tumor-specific antigens, viral DNA or RNA vaccine that expresses antigen in a living body being administered, and compositions containing cancer or tumor cells whose growth is suppressed and a crushed product or a lysate thereof are also included in "cancer vaccine". Furthermore, non-specific adjuvant such as bacterial cells, bacterial extracts and β-glucan, and oncolytic virus such as adenovirus are also included in "cancer vaccine". Normally, cancer vaccine contains an adjuvant for enhancing the specific immune response elicited by vaccine antigen. After being administered to a living body, vaccine antigen contained in cancer vaccine is phagocytosed by antigen-presenting cells (mainly dendritic cells and macrophages), processed inside the cells, turned into an epitope peptide consisting of amino acids of about 8 to 30 residues, and presented on the surface of antigen presenting cells as a complex bound with major histocompatibility complex (MHC) class I or class II. Regarding the length of the epitope peptide, it consists of amino acids of 8 to 11 residues in case of MHC class I and those of 13 to 30 residues in case of MHC class II. The T cell receptor (TCR) which expresses on the surface of cytotoxic T cells and helper T cells specifically recognizes MHC class I/peptide complex or MHC class II/peptide complex, respectively. As a result, these T cells are activated and exert antitumor effect. That is, cytotoxic T cells recognize and destroy cancer cells that present the same epitope peptide as that contained in vaccine antigen. Helper T cells enhance the action of cytotoxic T cells through secretion of cytokines and chemokines such as interferon (IFN)-y and interleukin (IL)-2, etc. Helper T cells also enhance antigen presenting ability of antigen presenting cells via CD40 ligand (CD40L) / CD40 pathway and production of antigen-specific immunoglobulin (Ig) G antibody by B cells.

In the present invention, the T cells produced by the method refer to cells which express antigen receptors called T cell receptors (TCR) on cell surface.

The antigen-specific T cells of the present invention can act on any antigen or tissue. In particular, when the T cells of the present invention are used in T cell supplement therapy, it is preferable that the T cells collected are specific to a cancer or tumor to be treated, tumor-related antigen or its variant antigen, or viral antigen, etc.

Examples of the tumor-related antigen or its variant antigen include, but are not limited thereto, those that express specifically or non-specifically in each tumor, such as WT1, GPC3, XAGE1, MUC1, MUC5AC, MUC6, EGFRvIII, HER-2/neu, MAGE A3, MAGE A1, Telomerase, PRAME, SSX2/4, PSCA, CTLA-4, gp100, GD2, GD3, Fucosyl GM1, GM3 , SLe (a), glycolipid F77, mesothelin, PD-L1, trp1, trp2, CD19, CD20, CD22, ROR1, CD33, c-Met, p53, p53 mutant, NY-ESO-1, PSMA, ETV6-AML, CEA, PSA, AFP, hTERT, EpCAM, ALK, androgen receptor, EphA2, CYP1B1, OY-TES-1, MAD-CT-2, MelanA/MART1, survivin, Ras, Ras mutant, ERG, bcr- abl, XBP1, and tumor-related antigen such as novel tumor antigen (neoantigen) caused by gene mutations and splice abnormalities. In addition, examples of viral antigen include, but are not limited thereto, inactivated virus such as inactivated HBV and HPV, and protein derived from various viruses, such as EBV LMP1, EBV LMP2, EBNA (EBV nuclear antigen), HPV E1, HPV E2, HPVE6, HPV E7, HBV HBs, HBV HBc, HBV HBe, HCV core, HCV NS3, HCV NS3/4A, HCV E1, HCV E2 , HCV NS5A, HIV gag, HIV pol, HIV nef, HIV env, CMV pp65, Influenza M1, HTLV-1 Ta and HBZ (HTLV-1 bZIP factor). Further, in case of protein antigen, peptide fragment thereof may also be used.

In an embodiment of the present invention, the production of T cells comprises a step of separating T cells that have been activated and amplified by contacting with one or more tumor-related antigens or viral antigens. When T cells are contacted with tumor-related antigen or viral antigen, T cell receptors receive the recognition and costimulatory signals of the tumor-related antigen or viral antigen, by which T cells are activated. As a process of contact, for example, mononuclear cells in peripheral blood containing antigen-presenting cells and T cells are cultured using a culture medium added with tumor-related antigen or viral antigen, or a biological tissue containing tumor-related antigen or viral antigen, or the crushed material or a lysate thereof. By adding cytotoxic inhibitor to the culture medium, the targeted T cells can be amplified efficiently.

Examples of cytotoxic inhibitor used in the present invention include, but are not limited thereto, perforin inhibitor (for example, Concanamycin A, SN34960, diarylthiophene, ethylene glycol bis (β-aminoethylether)-N, N, N', N'-tetraacetic acid (EGTA), ethylenediaminetetraacetic acid (EDTA), 1,2-bis-(o-aminophenoxy)-ethane-N,N,N',N'-tetraacetic acid (BAPTA), N-(2-hydroxyethyl)ethylenediamine-N,N',N'-triacetic acid (HEDTA), nitrilotriacetic Acid (NTA)) and granzyme inhibitor (for example, VTI-1002, Z-AAD-CH2Cl, Granzyme B inhibitor I, Granzyme B inhibitor II, Granzyme B inhibitor IV, Caspase-8 inhibitor I, Cell-Permeable, Caspase-8 inhibitor II).

In the present invention, the concentration of cytotoxic inhibitor added to the culture medium is 0.1 µM to 100 µM, preferably 0.5 µM to 50 µM, and more preferably 1 µM to 5 µM. After being added to the culture medium, it takes some time for cytotoxic inhibitor to diffuse and incorporate into cells, before exerting its maximum effect. It is desirable to add cytotoxic inhibitor to a culture medium, for example, 1 to 2 hours before the stimulation of T cells. After 1 to 2 hours, T cells can be activated while inhibiting cytotoxicity by adding T cell stimulant such as peptide antigen to a culture medium.

Cytotoxic T cells eliminate virus-infected cells and cancerous cells by recognizing antigen presented by HLA class I molecules and showing specific cytotoxicity. In-vitro amplification of antigen-specific T cells requires stimulation with target-antigenic peptide or protein. In particular, in case of amplification culture of T cells with regard to peripheral blood, the antigenic peptide or protein added are presented as antigen by antigen-presenting cells such as dendritic cells, and send activation signals to the T cells, by which the T cells activated specifically by antigens can be amplified. The HLA class I molecules used for antigen presentation are expressed in-vivo in almost all cells, and T cells themselves are also expressed.

Also, when amplification of specific T cells is carried out using antigenic peptide or protein, cytotoxicity of the antigen-presenting cells is expected to occur at the same time of T cell activation. When there are a few antigen-presenting cells, antigen presentation occurs between T cells, by which cytotoxicity between T cells is expected to occur.

According to the present invention, by inhibiting the cytotoxicity between T cells, the efficiency of the amplification of specific T cells was improved without interfering with the mechanism of activating and amplifying T cells by stimulation using the target antigenic peptide or protein. In addition, during T cell amplification culture using antigenic peptide or protein, cytotoxicity between T cells can be suppressed by adding Concanamycin A, a perforin inhibitor, without interfering with the activation and amplification of the T cells.

In an embodiment of the present invention, it is preferable that Concanamycin A, a perforin inhibitor, is added to a culture medium. By inhibiting the cytotoxicity between T cells, the efficiency of the amplification of specific T cells was improved without interfering with the mechanism of activating and amplifying T cells by stimulation using the target-antigenic peptide or protein. In addition, during T cell amplification culture using antigenic peptide or protein, cytotoxicity between T cells can be suppressed by adding Concanamycin A, a perforin inhibitor, without interfering with the activation and amplification of the T cells.

Activated T cells express functional molecules such as CD137 molecule and IFN-γ on the surface of cell membrane. Target antigen-specific T cells can be produced by staining these functional molecules with specific antibodies labeled with fluorescent proteins or magnetic beads, and sorting using a flow cytometer or separating using a magnet.

In the present invention, the "antigen-specific" means that T cells show an immune reaction to target tissue or antigen, while specifically recognizing an antigen epitope or peptide sequence.

In an embodiment of the present invention, as a method for producing target antigen-specific T cells, for example, a method of contacting antigenic protein or peptide with T cells to activate the T cells and separating the amplified T cells can be mentioned. In this method, mononuclear cells fraction containing antigen-presenting cells are cultured, and antigenic protein or peptide is added to the culture medium. By repeatedly contacting the antigenic protein or peptide with T cells, the reactive T cells proliferate and their percentage to the total T cells increase. On the other hand, the T cells that do not react with the antigenic protein or peptide added are not activated. They decrease gradually in percentage during in-vitro culture, and eventually die. Accordingly, after culturing for a certain period of time, T cells having specific reactivity with the antigenic protein or peptide added to the culture medium are isolated and obtained.

In an embodiment of the present invention, examples of methods for producing T cells include a method of selecting T cells that express CD137 and a method of selecting T cells that produce IFN-γ after activating T cells. In these methods, after binding an antibody specific for CD137 or IFN-γ, it can be subjected to magnetic separation using magnetic beads or selective separation by flow cytometer using fluorescent label. A system of capturing cells that produce IFN-γ with bispecific antibody (bispecific antibody against T cells and IFN-γ) may also be used. Furthermore, in another embodiment of the present invention, a method of selecting T cells to which an oligomer of antigenic peptide-HLA (human leukocyte antigen) complex binds can also be mentioned. In this method, target antigen-specific T cells can be obtained by labeling the oligomer consisting of a complex of target-antigenic peptide which specific HLA type and its HLA can present with a fluorescent dye and sorting the cells to which the oligomer bounds using a flow cytometer. These methods may be used alone or in combination when producing the T cells.

In an embodiment of the present invention, the degree of antigen specificity is preferably 30% or more, more preferably 40% or more, and still more preferably 50% or more of antigen-specific T cells as the starting material.

In an embodiment of the present invention, the T cells produced are preferably used in T cell supplement therapy. In the present invention, the T cell supplement therapy means a therapeutic method for replenishing T cells to a living body. In an embodiment of the present invention, preferable examples of T cell supplement therapy include therapeutic methods by cell transplantation such as infusion, intratumoral injection, intraarterial injection, and portal vein injection, etc. of T cells.

When the T cells obtained according to the present invention are used in T cell supplement therapy, in case of an allogeneic transplantation, from the viewpoint of difficulty to cause rejection, it is preferable that a cancer or non-cancer patient from whom T cells are collected and a cancer patient to whom the T cells obtained according to the present invention are administered for cancer treatment have the same HLA type. In addition, it is more preferable that a cancer patient to whom the T cells obtained according to the present invention are administered and a cancer patient from whom the T cells are collected are the same person. That is, cancer treatment based on autologous transplantation is more preferable.

In the pharmaceutical composition containing the T cells of the present invention, the percentage of the number of antigen-specific T cells to the total number of T cells is preferably 10% or more, more preferably 20% or more, and most preferably 30% or more.

In the pharmaceutical composition containing the T cells of the present invention, the number of antigen-specific T cells is preferably 1 × 10³ or more, more preferably 1 × 10⁴ or more, and most preferably 1 × 10⁵ or more.

The pharmaceutical composition of the present invention may contain pharmaceutically acceptable additives. Examples of the additives include cell culture medium, phosphate buffered saline and the like.

The pharmaceutical composition containing the T cells of the present invention can be used for preventing or treating cancer or viral infection. In an embodiment of the present invention, a method for preventing or treating cancer or viral infection using the pharmaceutical composition of the present invention is included.

Examples of cancer include, but are not limited thereto, ovarian cancer, hepatoblastoma, liver cancer, gastric cancer, esophageal cancer, pancreatic cancer, renal cell cancer, breast cancer, malignant melanoma, non-small cell lung cancer, cervical cancer, glioblastoma, prostate cancer, neuroblastoma, chronic lymphocytic leukemia, papillary thyroid cancer, colon cancer, B-cell non-Hodgkin lymphoma, and the like.

Examples of viral infection include, but are not limited thereto, hepatitis B virus, human papilloma virus, hepatitis C virus, EB virus, human immunodeficiency virus (HIV), human cytomegalovirus (CMV), influenza virus, human T-cell leukemia virus (HTLV), and the like.

### EXAMPLES

Next, the present invention will be described in more details with reference to the example. However, the scope of the present invention is not limited thereby.

### Example 1

Re-inoculate in a 24-well plate. Add 0.3 to 3 [µM Concanamycin A to a medium and incubate at 37°C and 5% CO₂ for 2 hours. Add 10 µg/ml EBV LMP2A peptide and culture at 37°C and 5% CO₂ for 24 hours. Suspend 2x10⁶ cells of peripheral blood mononuclear cells (PBMC) separated from peripheral blood of a healthy subject in a MEM-alpha medium added with 10% FBS, 200 U IL-2 and 20 µg/ml IL-15, and add 10 µg/ml EBV LMP2A peptide. Start culturing at 37°C and 5% CO₂. Replace the medium and divide the wells every 2 to 5 days according to amplification rate of the cells. Culture for 12 days. Collect the cells on day 12 and re-inoculate in a 24-well plate at 2x10⁶ cells/well. Add 0.3 to 3 µM Concanamycin A to the medium and incubate at 37°C and 5% CO₂ for 2 hours. Then, add 10 µg/ml EBV LMP2A peptide and culture at 37°C and 5% CO₂ for 24 hours. After 24 hours, stain the cells with CD8-PerCP/Cy5.5 antibody and CD137-PE antibody, and analyze using a flow cytometer (BD FACS Aria II).

As described above, the present invention has been described according to its embodiments. However, it should be understood that the description and drawings forming parts of the disclosure do not limit the present invention. From the disclosure, various alternative embodiments, examples and operational techniques will be apparent to those skilled in the art. The technical scope of the present invention is defined only by the matters for specifying the invention according to the scope of claims appropriate from the above description, and can be embodied at an implementation stage by modifying within the scope not departing from its gist.

## Claims

1. A method for producing antigen-specific T cells, comprising a step of stimulating mononuclear cells in a medium containing cytotoxic inhibitor, wherein the cytotoxic inhibitor is perforin inhibitor or granzyme inhibitor, preferably, perforin inhibitor is one or more selected from the group consisting of Concanamycin A, SN34960, diarylthiophene, ethylene glycol bis (β-aminoethylether)- N,N,N',N'-tetraacetic acid (EGTA), ethylenediaminetetraacetic acid (EDTA), 1,2-bis-(o-aminophenoxy)-ethane-N,N,N',N'-tetraacetic acid (BAPTA), N-(2-hydroxyethyl)ethylenediamine-N,N',N'-triacetic acid (HEDTA) and nitrilotriacetic acid (NTA), more preferably, the perforin inhibitor is Concanamycin A.

2. The method according to claim 1, wherein the mononuclear cells are lymphocyte.

3. The method according to any one of claims 1 to 2, wherein the mononuclear cells are isolated from peripheral blood or tumor.

4. The method according to any one of claims 1 to 3, wherein the antigen is tumor-related or variant antigen, or viral antigen, preferably the tumor-related or variant antigen is peptide cancer antigen, and the viral antigen is inactivated HBV or its virus-derived protein, or inactivated HPV or its virus-derived protein.

5. The method according to any one of claims 1 to 4, wherein the mononuclear cells are stimulated with antigen.

6. The method according to any one of claims 1 to 5, wherein the antigen is one or more peptide cancer antigens selected from the group consisting of GPC3, WT1, XAGE1, LMP2 and neoantigen, or one or more viral antigens selected from the group consisting of EBV LMP1, EBV LMP2, EBNA (EBV nuclear antigen), HPV E1, HPV E2, HPV E6, HPV E7, HBV HBs, HBV HBc, HBV HBe, HCV core, HCV NS3, HCV NS3/4A, HCV E1, HCV E2 , HCV NS5A, HIV gag, HIV pol, HIV nef, HIV env, CMV pp65, Influenza M1, HTLV-1 Ta and HBZ.

7. Antigen-specific T cells obtained by the method according to any one of claims 1 to 6.

8. A pharmaceutical composition comprising the antigen-specific T cells according to claim 7.

9. The pharmaceutical composition according to claim 8 for preventing or treating cancer or viral infection, wherein the cancer is selected from the group consisting of ovarian cancer, hepatoblastoma, liver cancer, gastric cancer, esophageal cancer, pancreatic cancer, renal cell cancer, breast cancer, malignant melanoma, non-small cell lung cancer, cervical cancer, glioblastoma, prostate cancer, neuroblastoma, chronic lymphocytic leukemia, papillary thyroid cancer, colon cancer, and B-cell non-Hodgkin lymphoma, and the viral infection is selected from the group consisting of hepatitis B virus, human papilloma virus, hepatitis C virus, EB virus, human immunodeficiency virus (HIV), human cytomegalovirus (CMV), influenza virus and human T-cell leukemia virus (HTLV).

10. A kit for preventing or treating cancer or viral infection, which comprises the pharmaceutical composition according to any one of claims 8 and 9.

11. A preventive or therapeutic agent for cancer or viral infection, which comprises the pharmaceutical composition according to any one of claims 8 and 9.

12. A pharmaceutical composition comprising antigen-specific T cells, wherein the T cells are (i) CD3 and CD45 positive, (ii) proliferative, and (iii) could release IFN-γ and Granzyme B upon activation by antigen stimulation.

13. The pharmaceutical composition according to claim 12, wherein the percentage of the number of antigen-specific T cells to the total number of T cells in the pharmaceutical composition is 10% or more.

14. The pharmaceutical composition according to claim 13, wherein the number of the antigen-specific T cells is 1 × 10³ or more.
